# EUROPEAN PATENT SPECIFICATION

(11) **EP 4 208 239 B1**
(45) Date of publication and mention of the grant of the patent: **20.05.2026**
(21) Application number: 21864789.9
(22) Date of filing: 02.09.2021
(51) Int. Cl.: A61M 16/06, A61M 16/10, A62B 23/06, A61M 16/08

(54) **PATIENT INTERFACE FOR DELIVERY OF GAS**
PATIENTENSCHNITTSTELLE ZUR ABGABE VON GAS
INTERFACE PATIENT POUR L'ADMINISTRATION DE GAZ

(30) Priority: 04.09.2020 US 202063074642 P
(43) Date of publication of application: 12.07.2023
(73) Proprietor: Fisher & Paykel Healthcare Limited, East Tamaki Auckland 2013 (NZ)
(72) Inventor: GADSBY, Georgia, East Tamaki Auckland, 2013 (NZ); BAUMANN, Monika, East Tamaki Auckland, 2013 (NZ); SPENCE, Callum James Thomas, East Tamaki Auckland, 2013 (NZ); RONAYNE, Michael Paul, East Tamaki Auckland, 2013 (NZ); MONRO, Rory Alexander, East Tamaki Auckland, 2013 (NZ)
(74) Representative: Forresters IP LLP
(86) International application number: PCT/NZ2021/050158
(87) International publication number: WO 2022/050852

(56) References cited:
- US-A- 1 431 177
- US-A1- 2002 029 004
- US-A1- 2008 314 391
- US-A1- 2011 073 116
- US-A1- 2011 125 052
- US-A1- 2014 326 244
- US-A1- 2014 326 244
- US-A1- 2016 367 782
- US-A1- 2017 224 943
- US-A1- 2018 304 036
- US-A1- 2019 134 342
- US-A1- 2020 197 644
- US-A1- 2020 238 037
- US-A1- 2020 238 037
- US-A9- 2014 166 021
- US-B1- 6 478 026

## Description

### Technical Field

The present disclosure relates to a patient interface for delivery of gas to a patient. It relates particularly, but not exclusively, to an interface for directing or treating expiratory gases from the patient.

### Background

In various medical procedures respiratory support is required to achieve adequate gas exchange in the patient. Respiratory support involves delivery of respiratory gas to the patient's airway via a patient interface such as a nasal cannula, endotracheal tube (ETT), tracheostomy tube, laryngeal mask (LMA), nasal mask, face mask or the like. Respiratory support may be provided in an apnoeic patient, or in a patient who is spontaneously breathing. In many cases, it is desirable to provide respiratory gas to the patient at high flow rates to achieve effective oxygenation and to clear CO₂, reducing respiratory dead space.

Patients receiving respiratory support are typically very unwell and delivery of respiratory support is a significant factor in prospects and rates of recovery. Therefore, advances in respiratory support delivery systems and patient interfaces have focussed on features for safe and effective delivery of respiratory gas to the patient with little attention paid to management of expiratory gases. The following documents also disclose different patient interfaces with physical structures, namely US 2020/238037 A1, US 2014/326244 A1, US 2017/224943 A1, US 2011/125052 A1 and US 2011/073116 A1.

The discussion of the background included herein including reference to documents, acts, materials, devices, articles explain the context of the present disclosure. This is not to be taken as an admission or a suggestion that any of the material referred to was published, known or part of the common general knowledge in Australia or in any other country as at the priority date of any of the claims.

### Summary

The invention is defined in the appended claims.
Numerous respiratory illnesses are known to be transmitted through infected droplets of respiratory gases entering the respiratory system of uninfected subjects. The 2009 "Swine flu" A(H1N1) began in Mexico and rapidly spread to over 200 countries and territories. Coronaviruses form a large family of viruses that can cause a range of illnesses from the common cold to more serious diseases. SARS (severe acute respiratory syndrome), MERS (Middle East respiratory syndrome) and the more recent respiratory syndrome coronavirus 2 (SARS-CoV-2) which is responsible for causing the disease COVID-19 have reached pandemic status due to their high rates of transmission. According to the World Health Organisation (WHO) as at the priority date of this application, the transmission of severe acute SARS-CoV-2 is thought to occur primarily via droplets and fomites between an infector and infectee. However airborne spread of this and other respiratory illnesses has not been ruled out and it can be envisaged if certain aerosol-generating procedures are conducted on infected patients in health care facilities, dispersal of aerosols, including those in exhaled gases, can present a risk of carrying and spreading such respiratory illnesses.

Aerosols, being a suspension of tiny particles or droplets in the air, can be generated through various mechanisms, including the interaction of a high velocity air stream with that of a relatively slow-moving flow of liquid. These tiny particles or droplets can contain substances and/or contaminants that are undesirable for persons to come in contact with. The aerosols may be infective and/or contain pathogens and, if inhaled or contacted with fluids from the eye, may cause adverse health effects. Management of expiratory gases, which can contain such aerosols, can be challenging. Management of expiratory gases has the potential to become particularly challenging during delivery of high flow therapy with a non-sealing patient interface, such as nasal high flow (NHF) therapy, where respiratory gas is jetted into the patient's airway at flow rates of up to 70 L/min. There may be a perception that delivery of gas in this way contributes to or causes an aerosol generating procedure. This may in turn result in health care workers taking aerosol generating procedure precautions which can alter patient care.

The risk of transmission of infection depends on the quantity of viable pathogen that makes its way to a site of infection in the infectee, which in turn depends on the quantity of pathogen emitted through expiratory gases and the survival rate of the pathogen during transmission. Similarly, the risk of exposure to substances or contaminants, such as nebulized drugs, in a patient's expiratory gases depends on the quantity of drug that makes its way to an unintended recipient, which in turn depends on the quantity of drug emitted through respiratory gases, and the viability of the drug in the unintended recipient.

It would be desirable to mitigate one or more risks and/or perceived risks, associated with expiratory gases from patients receiving respiratory support.

Viewed from one aspect, the present disclosure provides a patient interface for delivery of gas to a patient, the patient interface comprising: at least one inspiratory element for directing a flow of gas to a patient airway, each inspiratory element comprising at least one inspiratory lumen through which said flow of gas is directed; at least one expiratory element comprising an expiratory gas flow path for directing expiratory gases from the patient; and at least one gas permeable body in the expiratory gas flow path; wherein the gas permeable body is configured such that expiratory gases in the expiratory gas flow path are directed through the gas permeable body before exiting the patient interface.

Typically, directing expiratory gases through the gas permeable body at least partially treats the expiratory gases before exiting the patient interface. Treating the expiratory gases may comprise mitigating release and/or reducing velocity of aerosols or pathogens or other contaminants in the expiratory gases from the patient interface ideally without significantly increasing resistance to flow. The gas permeable body may be any material suitable or configurable to limit travel of liquid through the body and/or to limit travel of aerosols through the body. The gas permeable body may comprise a material that is permeable to water molecules and gas flow and may be substantially impermeable to bulk flow of liquid water. In some embodiments, the gas permeable body may comprise an absorbent material, and/or may comprise one or more of polyester, polyurethane, nylon, polyethylene or a composite thereof, and/or a fabric and/or a polymer matrix fabric.

In some embodiments, the patient interface further comprises a securement device for positioning or securing the patient interface to the patient, retaining the interface in an operable position while gas is directed into the patient's airway. The securement device may comprise one or more of, for example, a headgear, head strap, ties, elastic, adhesive dermal pad or the like. In some embodiments, the securement device may comprise a cap/skull cap type headgear. The patient interface may comprise one or more connection features for coupling any of the securement devices listed. The one or more connection features may allow for permanent coupling of the securement device, or it may be removable or detachable.

In some embodiments, the patient interface further comprises a manifold body providing a gas inflow path to the inspiratory lumen from a respiratory gas source. The manifold body may be locatable upon the patient's face in an operable position and may be secured by a securement device. Alternatively the manifold body may be positioned off the patient's face such that it does not touch the patient's face, yet is operably positioned relative to the patient's face for achieving delivery of respiratory gas from the respiratory gas source to the inspiratory lumen of the patient interface. Typically, the manifold body is couplable with or comprises a gas delivery conduit to deliver respiratory gas from the respiratory gas source to the at least one inspiratory element, and is in fluid communication, via the inspiratory lumen, to the patient's airway e.g. via nasal elements such as nasal prongs of the patient interface.

In some embodiments, the patient interface comprises or is operatively couplable with one or more tubes for directing gas into the trachea of the patient. The patient interface may comprise a mask, tube or a nasal cannula comprising at least one nasal element (such as a nasal prong) configured to direct gas into a nare of the patient. It is to be understood that while some embodiments of the present disclosure may relate to a sealed patient interface in which there is sealing occlusion at the expiratory element, many embodiments relate to an unsealed interface where there is no sealing occlusion at the expiratory element.

In some embodiments, a nasal element of the patient interface provides for directing a flow of gas into a nare of the patient, with expiratory gases being released from the patient either orally, or around the nasal element but not through it. In such an arrangement, treatment of expiratory gases in the nasal expiratory flow path is achieved by the at least one gas permeable body being configured as a cover over at least part of the nasal element. The cover may be removable and/or replaceable on the nasal element and is ideally, shaped to non-sealingly occlude the nare in the sense that there is no direct line of sight into the nare, but there is flow of expiratory gas through the gas permeable body. In some embodiments, one or both of the nasal element and the cover occupy the nare such that expiratory gases from the nare are at least predominantly, if not entirely, directed through the gas permeable body.

In some embodiments, a nasal element of the patient interface further comprises one or more expiratory flow lumens and the patient interface comprises a manifold body providing a gas outflow path for expiratory gases from the expiratory flow lumens in the nasal elements to the at least one gas permeable body. In this arrangement, the gas permeable body may be provided in or form part or all of the manifold body. The nasal element containing an expiratory flow lumen need not seal the nare since at least a portion of the expiratory gasses will be treated by the gas permeable body in the manifold body. However, such an arrangement may further comprise a cover comprising a gas permeable body around the nasal element to provide treatment of expiratory gases released around the nasal element, as well as through the expiratory flow lumen in the nasal element.

In some embodiments, the patient interface further comprises a gas delivery conduit for delivering respiratory gas to the patient. The gas permeable body comprises a sheath around at least part of the gas delivery conduit and is configured to be in fluid communication with expiratory gases from the expiratory element. This may be achieved by an expiratory flow lumen through a manifold body providing an expiratory gas flow path from the nasal elements to the gas permeable sheath.

The gas permeable body may comprise a filter material selected from a group comprising but not limited to fibrous, knitted, woven, foam, textile, non-textile or other filter material. In some embodiments, the gas permeable body is configured to intercept aerosols in the expiratory gases having a size less than about 140 µm and preferably, less than about 120 µm although any filter material intercepting aerosols and/or other contaminants or particles such as viral or bacterial particles may be utilised provided they do not adversely interfere with delivery of therapy. By way of non-limiting example, some potentially airborne particles include biological particles of around 0.5 µm and some coronavirus particles of around 0.1 µm in size. Thus for some applications it may be ideal for the filter material of the gas permeable body to have a pore size capable of intercepting or at least slowing the velocity of such particles, without being so small that gas flow is negatively impacted For some applications, the gas permeable body may be provided by use of known filter material meeting e.g. N95, N99 or high-efficiency particulate air (HEPA) classification.

In some embodiments, the gas permeable body is configured to be dampened before use. Pre-wetting may improve capacity of the gas permeable body to attract, retain and/or trap aerosols. Additionally or alternatively, a heating source may be provided to deliver heat to the gas permeable body to prevent or reduce saturation of the gas permeable body to mitigate potential for clogging and/or contamination of the wet part. In some embodiments, heating may be provided to avoid saturation or to avoid wetting of the gas permeable body, which may increase resistance to flow.

In some embodiments comprising at least one nasal element configured to direct gas into a nare of the patient, the patient interface further comprises a plurality of physical structures on an external surface of the at least one nasal element, the physical structures being configured to intercept and/or slow aerosols in the expiratory gases from the at least one nare, e.g. by providing a tortuous expiratory gas flow path.

In some embodiments, the physical structures extend substantially radially from the surface of the one or more nasal elements. The physical structures may be or include a portion that is oriented at an acute angle relative to an outer surface of the nasal element in a direction of insertion into the nare, to facilitate insertion. The acute angle may be in a range of about 10 to about 80 degrees although a more orthogonal arrangement may be adopted, particularly when at least a plurality of the physical structures have some capacity for bending and/or pivoting at the base. It is also contemplated that the physical structures may consist of or comprise a portion that is oriented at an obtuse angle relative to the outer surface of the nasal element in a direction of insertion into the nare, and flex, or pivot at the base, to provide for insertion (and removal).

At least a plurality of the physical structures may have a shape or configuration that at least partly defines a tortuous path to intercept and/or slow aerosols in the expiratory gases. In some embodiments, the shape is selected from a group comprising but not limited to zig-zag, crenelated, coiled and serpentine. Alternatively/additionally at least a plurality of the physical structures may have a cross-sectional shape selected from a group comprising but not limited to chevron, vaulted, V-shaped, W-shaped, roughened, substantially cornette-shaped, rectangular, triangular and other regular or irregular closed polygonal shape. Alternatively or additionally, the physical structures themselves may have protrusions, for example they may be "hairy", the hairs assisting with capture or slowing of aerosols. The protrusions/hairs increase the surface area of the physical structures, increasing the likelihood of interception of aerosols and/or slowing their exit velocity. The physical structures may be solid, or they may be hollow or include a hollow portion which, depending on their composition, may add to the filtration performance of the structures and/or provide capillary action to draw moisture into the structures themselves.

In some embodiments, some or all of the physical structures have a coating and/or surface modification to improve performance. Coatings that make the physical structures sticky or electrostatic may increase the number of surfaces that intercepted droplets may condense on. Chemical and/or physical changes to the structure or microstructure of the physical structures, or utilising an absorbent material may improve performance. Such changes may include e.g. applying a hydrophilic coating to the physical structures. Surface modifications may increase adhesive capability of the physical structure, increasing the likelihood of droplets gathering.

In some embodiments, one or more of the physical structures has at least one end attached to the nasal element and may have both ends attached to the nasal element forming a loop structure. In some embodiments, mid sections may also be attached to the nasal element forming further loops.

In some embodiments, one or more of the physical structures is flexible and may comprise a pivotable or flexible portion at or towards the attached end to accommodate different nostril sizes by flexing or bending when in situ, and to facilitate insertion and removal from the patient's nare. The one or more physical structures may be resilient such that they can elastically deform when the nasal element is inserted into the nares of a patient.

In some embodiments the physical structures are provided in a substantially staggered or offset arrangement on the surface of the nasal element to maximise interception of aerosols in expiratory gases from the patient. It is to be understood, however, that a random or substantially random arrangement may be adopted, as may any arrangement that reduces the line of sight past the physical structures and into the nare when in situ.

The physical structures may take a variety of different configurations and combinations of configurations. In some embodiments, one or more of the physical structures comprise filaments or whiskers. In certain configurations, the filaments or whiskers are arranged in an angled and staggered arrangement which resembles a conifer or "Christmas tree" like shape. The rows may concentric or they may be defined by a corkscrew arrangement around the nasal element. In some arrangements, the filaments or whiskers, or the physical structures more generally, are configured in a cascading arrangement of gradually increasing length progressing from shorter structures closer to the tip of the nasal element (inserted furthest into the nare) to longer structures toward the base of the nasal element, or vice versa. In other arrangements, the physical structures all have the same length and in yet other arrangements, the physical structures may have various lengths which may be substantially random or arranged to suit the internal contours of the nare.

In some embodiments, one or more of the physical structures comprise fins or pleats. Pleats may be formed by pinching or folding into pleats one or more lengths of a material arranged around the nasal element, increasing the surface area of the material around the nasal element. In other embodiments, the physical structures may be configured as a ruff or relatively stiff frill around the nasal element. In some embodiments, fins or pleats are angled to intercept and redirect flow of expiratory gases. The fins or pleats may be oriented on the surface of the nasal element such that they extend radially but need not be orthogonal to the surface. They may be arranged uniformly, equidistant, at uniform angles or at differing spacing and angular orientation with respect to each other and the surface of the nasal element. There may be a single layer of fins or pleats provided around the nasal element. Alternatively, there may be several rows of fins or pleats provided around the nasal element, which may be offset to reduce the direct line of sight past the fins/pleats and into the nare when in situ.

In some embodiments, at least a plurality of the physical structures are hydrophilic to attract droplets in the expiratory gases, or are coated with a hydrophilic material.

The physical structures may be applied directly to the surface of the nasal element or they may be provided on a substrate configured to be applied to an external surface of the nasal element e.g. by use of an adhesive. Alternatively/additionally, the substrate may comprise a tube-shaped sleeve fixable over the nasal element.

In some embodiments, the patient interface further comprises at least one nasal element (such as a nasal prong) configured to direct gas into a nare of the patient, the at least one nasal element being formed at least in part from material comprising the gas permeable body. In this arrangement, the nasal element may further comprise an internal membrane defining the inspiratory lumen within the nasal element.

In some embodiments, the patient interface further comprises at least one nasal element (such as a nasal prong) configured to direct gas into a nare of the patient, wherein the gas permeable body comprises a substantially cylindrical body positioned around the nasal element. The gas permeable body may comprise an open cell foam or mesh, porous or fibre filter materials such as those formed from plastics, fabrics, fibres, fibreglass, synthetic polymer fibres, fabrics e.g. polypropylene fabric; foams (expanded polyethylene, polyurethane, silicone, rubber, etc) and the like.

In some embodiments, the patient interface further comprises a channel for collection of droplets removed from expiratory gases. The channel may be configured to direct collected droplets to return to the patient during treatment. Ideally, collected droplets are directed by gravity flow although this need not be the case, and droplets may be blown back, e.g. utilising flows from respiratory support, or utilise capillary action.

The physical structures may take a variety of different configurations and combinations of configurations. In some embodiments, one or more of the physical structures comprise filaments or whiskers. In certain configurations, the filaments or whiskers are arranged in an angled and staggered arrangement which resembles a conifer or "Christmas tree" like shape. The rows may concentric or they may be defined by a corkscrew arrangement around the nasal element. In some arrangements, the filaments or whiskers, or the physical structures more generally, are configured in a cascading arrangement of gradually increasing length progressing from shorter structures closer to the tip of the nasal element (inserted furthest into the nare) to longer structures toward the base of the nasal element. In other arrangements, the physical structures all have the same length and in yet other arrangements, the physical structures may have various lengths which may be substantially random or arranged to suit the internal contours of the nare.

In some embodiments, one or more of the physical structures comprise fins or pleats. Pleats may be formed by pinching or folding into pleats one or more lengths of a material arranged around the nasal element, increasing the surface area of the material around the nasal element. In other embodiments, the physical structures may be configured as a ruff or relatively stiff frill around the nasal element. The fins or pleats may be angled to intercept and redirect flow of expiratory gases. The fins or pleats may be oriented on the surface of the nasal element such that they extend radially but need not be orthogonal to the surface. They may be arranged uniformly, equidistant, at uniform angles or at differing spacing and angular orientation with respect to each other and the surface of the nasal element. There may be a single layer of fins or pleats provided around the nasal element. Alternatively, there may be several rows of fins or pleats provided around the nasal element, which may be offset to reduce direct line of sight past the fins/pleats and into the nare when in situ.

In some embodiments, at least a plurality of the physical structures are hydrophilic or are coated with a hydrophilic material.

The physical structures may be applied directly to the surface of the nasal element or they may be provided on a substrate configured to be applied to an external surface of the nasal element. The substrate may have an adhesive backing for attachment of the substrate to the nasal element. Alternatively/additionally, the substrate may comprise a tube-shaped sleeve which is fixable, preferably removably fixable or replaceable, over the nasal element.

In some embodiments, the patient interface further comprises a channel for collection of droplets removed from expiratory gases. The channel may be configured to direct collected droplets to return to the patient during treatment. Ideally, collected droplets are directed by gravity flow although this need not be the case, and droplets may be blown back, e.g. utilising flows from respiratory support, or utilise capillary action.

In some embodiments, at least part of the patient interface is thermally conductive. One or more of the physical structures may be thermally conductive. Alternatively/additionally the inspiratory lumen may contain a thermally conductive material and is configured to facilitate warming of gases delivered to the patient such that there is thermal transfer to at least a plurality of the physical structures to enhance interception and/or slowing and/or reduction of momentum of aerosols or particles in the expiratory gases.

Viewed from another aspect, the present disclosure provides an accessory for a patient interface having a nasal element (such as a nasal prong) for delivery of gas to a patient, the accessory comprising a substrate supporting a plurality of physical structures, the substrate being applicable to an external surface of the nasal element. The substrate may be applied to an external surface of the nasal element e.g. by use of an adhesive. Alternatively, or additionally, the substrate may comprise a tube-shaped sleeve fixable over the nasal element.

In some embodiments, the physical structures are configured to intercept and/or slow aerosols in expiratory gases from the at least one nare. The physical structures may therefore be configured to provide a tortuous expiratory gas flow path and ideally, are arranged to intercept and/or slow aerosols having a size less than about 140 µm and preferably, less than about 120 µm.

Viewed from another aspect, the present disclosure provides an accessory for a patient interface having a nasal element (such as a nasal prong) for delivery of gas to a patient, the accessory comprising a gas permeable body positionable around at least part of the nasal element, the gas permeable body being configured to treat expiratory gases passing therethrough before exiting the accessory. In some embodiments, the gas permeable body is substantially cylindrical to receive a nasal prong of the nasal element when in use. Alternatively/additionally the gas permeable body may be configured to substantially surround at least a base portion of prongs of the nasal element and optionally to occupy all or part of a space between the upper surface of a manifold portion of the patient interface and the patient's nares when in use.

In some embodiments, the gas permeable body comprises an open cell foam or mesh, porous or fibre filter materials such as those formed from plastics, fabrics, fibres, fibreglass, synthetic polymer fibres, fabrics e.g. polypropylene fabric; foams (expanded polyethylene, polyurethane, silicone, rubber, etc) and the like.

It is to be noted that any one of the aspects mentioned above may include any of the features of any of the other aspects and may include any of the features of any of the embodiments in the following sections.

It is to be understood each of the various aspects described herein may incorporate features, modifications and alternatives described in the context of one or more other aspects, such as but not limited to the various features of aspects of the accessory, such as the gas permeable body and the physical structures, which may incorporate any one or more of the various features of the gas permeable body and the physical structures as described in the context of the various aspects of the patient interface. For efficiency, such features, modifications and alternatives have not been repetitiously disclosed for each and every aspect although one of skill in the art will appreciate that such combinations of features, modifications and alternatives disclosed for some aspects apply similarly for other aspects and are within the scope of and form part of the subject matter of this disclosure.

### Brief Description of Drawings

The present disclosure will now be described in greater detail with reference to the accompanying drawings. It is to be understood that the embodiments shown are examples only and are not to be taken as limiting the scope of the disclosure.
Figure 1 is a schematic view of an overall respiratory assistance or support system.
Figure 2 is a schematic illustration of a patient interface having nasal elements comprising nasal prongs, with filtration provided by gas permeable covers over the nasal prongs.
Figure 3 is a schematic illustration of a patient interface having nasal elements with filtration provided by a gas permeable body provided in a manifold body.
Figure 4 is a schematic illustration of a patient interface having nasal elements with filtration provided by a gas permeable body remotely located from the nasal elements.
Figure 5 is a schematic illustration of a patient interface comprising a nasal mask with a gas permeable body.
Figure 6 is a schematic illustration of a patient interface comprising a full face mask with two gas permeable bodies.
Figure 7 is a schematic illustration of part of a patient interface comprising a nasal mask with a remotely located gas permeable body (not shown).
Figure 8 is a schematic illustration of a patient interface comprising nasal elements showing a plurality physical structures provided on their external surface in the form of filaments or whiskers.
Figure 9 is a schematic illustration of a nasal element showing a plurality of physical structures provided on the external surface according to another embodiment of the disclosure.
Figures 10a-c are schematic illustrations showing examples of cross-sectional shapes that may be incorporated into physical structures according to some embodiments of the disclosure.
Figure 11 is a schematic illustration of a patient interface comprising nasal elements showing a plurality of physical structures on their external surface in the form of fins.
Figure 12 is a schematic illustration of a patient interface comprising nasal elements, showing a gas permeable body in the form of a substantially cylindrical body positioned around each nasal element.
Figure 13 is a schematic illustration of a patient interface comprising a pair of nasal prongs and a porous body which substantially surrounds at least the base of the prongs.

### Detailed Description

Embodiments of the present disclosure are directed to a patient interface used in respiratory support which provides features for directing and/or treating expiratory gases from the patient. Treatment of expiratory gases may reduce transmission risk in circumstances where those gases may contain infective or pathogenic particles, as well as reducing risks associated with the presence of other substances, for example, nebulised drugs, in expiratory gases. Some embodiments of the disclosure are particularly directed to reducing transmission risk arising from aerosols in the expiratory gases from a patient, including expiratory gases from a patient receiving e.g. NHF therapy, although it is to be understood that the scope of this disclosure is not limited to such application. The present disclosure provides embodiments for intercepting/capturing and/or slowing the velocity of aerosols and other particles in expiratory gases to reduce the likelihood of those particles travelling a sufficient distance to either enter the airway of another person or land on a surface that could present a transfer risk when touched.

A key factor in determining if respiratory droplets pose a risk of becoming airborne is the size of the droplets. Respiratory droplets come in a range of sizes, characterized by diameters with an upper limit of several hundred µm. Droplets evaporate as a function of their size, speed, chemical composition and how they are clustered together in the expiratory 'jet'. These dependencies arise because evaporation is driven by a difference in partial pressure and temperature at the surface of the evaporating substance and its environment. External influences on the evaporation include the room temperature, humidity and ventilation patterns. Large droplets fall to the ground or another surface before they completely evaporate. These form the droplet route of infection. Smaller droplets evaporate to become so small (less than ~5 µm) that they linger for several minutes in the air. These are called droplet nuclei and represent the airborne route of infection.

Typically, droplets larger than ~120 µm fall to the ground quickly, posing a droplet transmission risk whereas droplets smaller than ~120 µm evaporate leaving a suspension of particles in the air and therefore pose an airborne transmission risk. Of course relative humidity has an effect with higher humidity environments slowing evaporation and lower humidity environments accelerating evaporation and therefore, airborne transmission risk.

Embodiments of the present disclosure provide a patient interface which may reduce the likelihood of pathogens and contaminants in the expiratory gas becoming airborne by physically filtering droplets through a gas permeable body, and/or by altering the flow of expiratory gases e.g. by creating a tortuous pathway for droplets to traverse. These features are aimed at reducing the likelihood of aerosolization of these particles and/or reducing the likelihood of spread by slowing the outflow velocity of unfiltered pathogens in the expiratory gases.

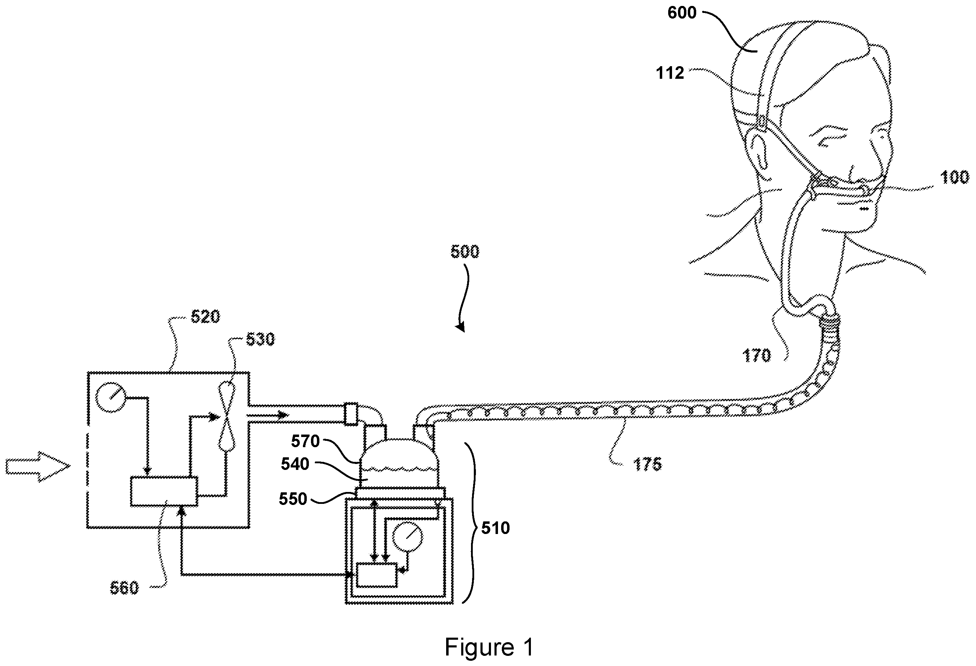

Referring initially to Figure 1, a respiratory support or assistance system 500 that may be used with the patient interfaces of the present disclosure is shown. In such a system, a patient 600 is supplied with a flow of gases through a patient interface 100. The flow of gases may be humidified. The patient interface 100 is retained in an operational position upon the patient's face using associated securement device 112, headgear or the like. The securement device 112 provides a retention mechanism configured to retain the patient interface 100 on the patient's face in use.

The patient interface 100 is connected to a humidified gases transportation pathway or inspiratory conduit 170. The inspiratory conduit 170 is connected at one end to the patient interface 100 and at an opposing end to an outlet of a humidifier 510. The inspiratory conduit 170 may be connected to the patient interface 100 via an extension tube 175. The extension tube 175 and/or the inspiratory conduit 170 form a patient conduit adapted to connect to a gases source for delivering a gases flow to the patient 600.

The extension tube 175 and/or conduit 170 can be part of the patient interface 100. Therefore, as used herein, "patient interface" can be not only the portion of the interface that contacts and/or interfaces with the patient, but also the conduit that delivers the flow of gases to the interface.

The humidifier 510 receives and humidifies gas supplied from a flow generator or respiratory gas source 520, which may include a blower 530. Alternatively or additionally, other gas sources may be used, such as a wall source with a flow meter. The humidifier 510 and gases source 520 may be integrated or may be in separate modules. The humidifier 510 may comprise a humidification chamber 570, filled with water or other liquid 540 and a heating means 550 for heating the water 540 to humidify the gas path through the humidifier 510. A controller 560 may be provided to control and possibly vary one or more properties of the supplied gas, including but not limited to the pressure profile of the gas, flow rate profiles of the gas at the patient interface 100, temperature and/or humidity of the gas. It will be appreciated that the control capabilities are dependent on the purpose and application of the respiratory support system 500.

The respiratory system 500 may be a high flow therapy apparatus or system. High flow therapy as discussed herein is intended to be given its typical ordinary meaning as understood by a person of skill in the art, which generally refers to a respiratory support system delivering a targeted flow of humidified respiratory gases via an intentionally unsealed patient interface with flow rates generally intended to meet or exceed inspiratory flow of a patient. Typical patient interfaces include, but are not limited to, a nasal or oral patient interface. Typical flow rates for adults often range from, but are not limited to, about fifteen litres per minute (LPM) to about seventy LPM or greater. Typical flow rates for paediatric patients (such as neonates, infants and children) often range from, but are not limited to, about one litre per minute per kilogram of patient weight to about three litres per minute per kilogram of patient weight or greater. High flow therapy can also optionally include gas mixture compositions including supplemental oxygen and/or administration of therapeutic medicaments. High flow therapy is often referred to as nasal high flow (NHF), humidified high flow nasal cannula (HHFNC), high flow nasal oxygen (HFNO) and high flow therapy (HFT) among other common names. The flow rates used to achieve 'high flow' may be any of the flow rates listed below.

For example, in some configurations, for an adult patient 'high flow therapy' may refer to the delivery of gases to a patient at a flow rate of greater than or equal to about 10 LPM, such as between about 10 LPM and about 120 LPM, or between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow therapy' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow therapy apparatus with an adult patient, a neonatal, infant, or child patient, may deliver gases to the patient at a flow rate of between about 1 LPM and about 100 LPM, or at a flow rate in any of the sub-ranges outlined above. The flow therapy apparatus 500 can deliver any concentration of oxygen (e.g., fraction of delivered oxygen, FdO₂), up to 100%, at any flowrate between about 1 LPM and about 100 LPM. In some configurations, any of the flowrates can be in combination with oxygen concentrations (FdO₂) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO₂) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 500 may include safety thresholds when operating in manual mode that prevent a user from delivering too much oxygen to the patient.

High flow therapy may be administered to the nares of a user and/or orally. High flow therapy may deliver gases to a user at a flow rate at or exceeding the intended user's peak inspiratory flow requirements. The high flow therapy may generate a flushing effect in the nasopharynx such that the anatomical dead space of the upper airways is flushed by the high incoming gases flow. This can create a reservoir of fresh gas available for each breath, while minimizing re-breathing of nitrogen and carbon dioxide. Meeting inspiratory demand and flushing the airways is additionally important when trying to control the patient's fraction of inhaled oxygen, FiO₂. High flow therapy can be delivered with a non-sealing patient interface such as, for example, a nasal cannula. The nasal cannula may be configured to deliver breathing gases to the nares of a user at a flow rate exceeding the intended user's peak inspiratory flow requirements.

The term 'non-sealing patient interface' as used herein can refer to an interface providing a pneumatic link between an airway of a patient and a gases flow source that does not completely occlude the airway of the patient. Non-sealed pneumatic links can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or both nares of the patient. For a nasal cannula the airway is through one or both of the nares.

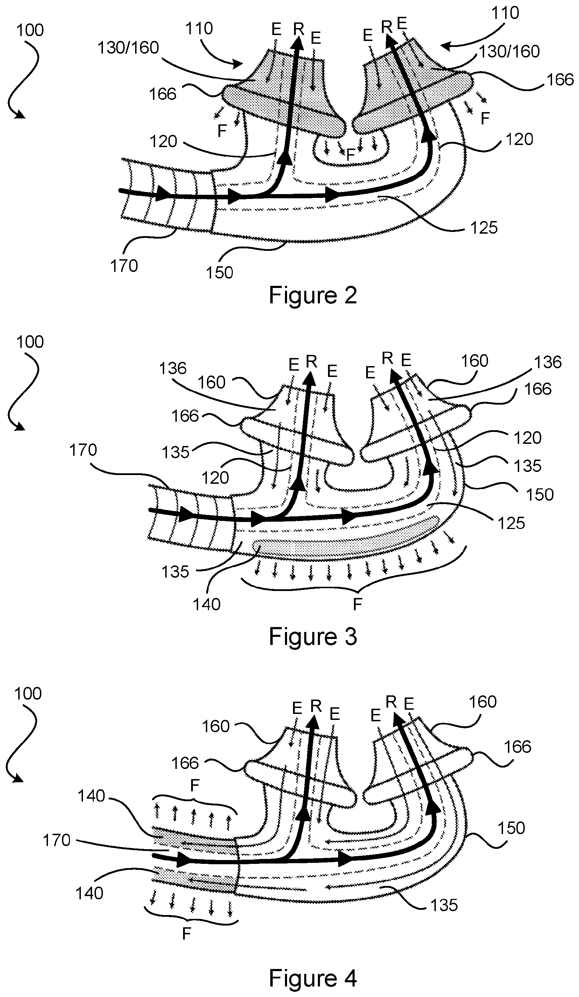

Referring to Figures 2 to 7, a patient interface 100 for delivery of gas to a patient 600 is shown. The patient interface 100 has an inspiratory element comprising an inspiratory lumen 120 for directing a flow of gas to a patient airway, via the nares. An expiratory element comprising an expiratory gas flow path 130 directs expiratory gases from the patient. Provided in the expiratory gas flow path is a gas permeable body 160. The gas permeable body 160 may be provided in any suitable location or component of patient interface 100 provided that it is configured such that expiratory gases in the expiratory flow path 130 are directed through the gas permeable body before exiting the patient interface, several examples of which are described in further detail below. Ideally the configuration is such that the gas permeable body 160 at least partially treats the expiratory gases before exiting the patient interface 100. In some embodiments, treating the expiratory gases may involve mitigating release and/or reducing velocity of aerosols or other contaminants into the ambient air.

Ideally, the patient interface 100 includes a means for mounting or otherwise attaching the apparatus on the patient. In some embodiments, a securement device 112 (Figure 1) is provided to secure the patient interface 100 to the patient and may include an elasticised or resilient strap, ties or an adhesive dermal pad for temporarily attaching at least part of the patient interface to the patient's skin thereby retaining the patient interface in an operable position. Alternatively, the securement device may comprise a cap/skull cap type headgear. Such headgear may comprise a plurality of straps or a continuous or meshed fabric or non-woven sheet forming a cap. Alternatively/additionally such headgear may comprise a combination of rigid or substantially rigid members and straps. The patient interface 100 may comprise one or more connection features for coupling any of the securement devices listed. The one or more connection features may allow for permanent coupling of the securement device, or it may be removable or detachable.

### Nasal cannula

In the embodiment of Figure 2, the expiratory gas flow path 130 of the expiratory element is provided through a cover 160 over the part of the nasal element that defines inspiratory lumen 120. In some embodiments, this part of the nasal element may be described as a nasal prong, and the cover 160 is in the form of a cap or pillow which is at least partly formed from the material that comprises the gas permeable body. While the embodiment shown provides cover 160 as a separate element, it is to be understood that the cover could be integral with or part of nasal the element. Ideally, the entirety of cover 160 is formed from the material that comprises the gas permeable body and is sized and/or shaped to occupy the nare such that expiratory gases E from the nare are at least predominantly directed through the gas permeable body, the external surface of which abuts the internal wall of the nare such that expiratory gases are directed through it. If the nare is not fully occupied by cover 160, some expiratory gas E may flow around the nasal element without being directed through the gas permeable body 140. However, a significant proportion of expiratory gas E will be directed through the gas permeable body and released as treated or filtered expiratory gas F, thereby reducing the velocity of expiratory gasses, and the likelihood of release of aerosols of a size likely to be or capable of becoming airborne compared with conventional patient interfaces that do not treat the expiratory gases at all.

In some embodiments, cover 160 may be removable from the nasal prongs and replaceable e.g. when gas permeable material of the cover becomes saturated with intercepted droplets or moisture from humidified respiratory gases. Advantageously, such covers may be applied to existing nasal cannulas to reduce risk of transmission arising from airborne droplets. Such covers may be provided in a range of sizes and materials which may be selected according to the clinical environment in which they are used. For example, for patients receiving therapy delivered by the nares for treatment of COVID-19, it may be desirable to utilise nasal prong covers 160 formed of or containing a gas permeable body comprising an N95 or higher (e.g. N99) rated or HEPA filter material to reduce transmission risk. Other suitable materials may include mesh, porous or fibre filter materials such as those formed from plastics, fabrics, fibres, fibreglass, synthetic polymer fibres, fabrics e.g. polypropylene fabric and open cell foams (such as expanded polyethylene, polyurethane, silicone, rubber, etc).

In some embodiments, cover 160 includes a skirt 166 configured to rest over the opening to the nare to improve occlusion and ensure substantially all expiratory gases E from the nare are directed through the gas permeable body.

Figure 2 also shows a manifold body 150 which provides a gas inflow path through manifold inspiratory lumen 125 to the inspiratory lumen 120 from a conduit 170 which is in fluid communication with a respiratory gas source. It is to be understood, however, that manifold body 150 may be omitted and the flow of respiratory gas R may be provided by conduit 170 directly connected or coupled with the inspiratory lumen 120 of the nasal prongs. Alternatively, cover 160 may provide inspiratory lumen 120 in lieu of nasal prongs, for delivery of respiratory gas from manifold body 150. In such arrangement, cover 160 has an internal membrane which defines inspiratory lumen 120 and is configured for coupling with manifold body 150 such that the inspiratory lumen in the cover is in fluid communication with manifold inspiratory lumen 125.

In the embodiment of Figure 3, the gas permeable body 140 is provided in manifold body 150 and expiratory gases E are directed from the nares through an expiratory gas flow path in the manifold body such that treated or filtered expiratory gases F are released from patient interface 100. In the embodiment shown, the expiratory gas flow path is defined by an expiratory lumen 136 in cover 160 which may be provided over the part of the nasal element (e.g. nasal prong) that defines inspiratory lumen 120. Alternatively, the nasal prong itself may provide both an inspiratory lumen (as described in relation to Figure 1) and an expiratory lumen and the expiratory lumen may be parallel or coaxial with the inspiratory lumen, and in fluid communication with respective inspiratory lumen 125 and expiratory lumen in manifold body 150. Cover 160 is configured for coupling with manifold body 150 such that the expiratory lumen 136 in the cover is in fluid communication with manifold expiratory lumen 135 which together form the expiratory gas flow path directing expiratory gases through gas permeable body 140 in manifold body 150 before exiting patient interface 100 as treated or filtered expiratory gases F.

Figure 3 shows gas permeable body 140 in a base portion of manifold body 150 and in fluid communication with manifold expiratory lumen 135. However, it is to be understood that the location of gas permeable body 140 in manifold body 150 is not to be limited in this way and may be provided in any aspect of the manifold body which provides fluid communication with manifold expiratory lumen 135. In some embodiments, the manifold body may be manufactured in its entirety, or in most part, from the material that comprises the gas permeable body 140. In this arrangement, expiratory gases from expiratory lumen 136 of cover 160 may be released through the manifold body itself via pores of the gas permeable material from which it is made. Alternatively, manifold expiratory lumen 135 may be provided as a bore through the manifold body 150 such that expiratory gasses from expiratory lumen 136 of cover 160 are released through those parts of the manifold body that are formed from or contain a gas permeable body 140.

In some embodiments of patient interface 100, gas permeable body 140 may be located more distally from the nasal elements. In the example shown in Figure 4, the gas permeable body 140 is configured as a sheath formed around at least part or all of gas delivery conduit 170. Manifold expiratory lumen 135 is in fluid communication with pores of gas permeable body 140 such that expiratory gases E are directed from the nares through manifold body 150 and then through gas permeable body 140 such that treated or filtered expiratory gases F are released from patient interface 100. Alternatively or additionally, gas permeable body 140 could be a component within gas delivery conduit 170 or integral with the tube from which it is formed. For instance, the walls of gas delivery conduit 170 may be formed from e.g. extruded from gas permeable material, with the internal respiratory gas delivery channel defined by an internal gas impermeable membrane which maintains separation of inspiratory and expiratory gases.

It is to be understood that the arrangement of Figures 3 and 4 may be combined to increase the surface area of the gas permeable body 140 in the patient interface 100 which may advantageously limit any increase to resistance to flow attributable to expiratory gases being directed through the gas permeable body.

Cover 160 may occupy the nare to limit or preclude release of expiratory gases which are not first passed through a gas permeable body. Thus, cover 160 may be formed in part or in its entirety from the material that comprises the gas permeable body (as in Figure 2), or it may be a silicon, foam or other member configured to be applied over the nasal prong so as to occlude or occupy the nare. In some embodiments, cover 160 includes a skirt 166 configured to rest over the opening to the nare to improve occlusion and ensure all expiratory gases from the nare are directed through the gas permeable body.

It is to be understood that while embodiments of the disclosure are described in the context of nasal cannula devices directing a flow of gas into both nares of the patient, the disclosure also contemplates a patient interface which directs flow into a single nare.

### Mask body

In other embodiments, gas permeable body 140 is provided in mask or sealing element 180 and expiratory gases E are directed from the nares (and optionally, the mouth) through an expiratory gas flow path in the mask 180 such that treated or filtered expiratory gases F are released from patient interface 100. Mask 180 may be integral with the nasal elements directing respiratory gas into the patient's airway, such that the mask and nasal elements are part of a single integrated product requiring no additional assembly. Although convenient, this arrangement may be more expensive with less flexible treatment options. Thus in other embodiments, mask 180 may operate in concert with a nasal cannula as a modular set which may be connected e.g. using clips or other fasteners, only when the mask component is required. Ideally, the clips or fasteners are releasable. Ideally, mask 180 provides a sealing portion which is configured to seal about or against the patient's face. The seal may comprise a cushion member configured to seal about or against the patient's face. The seal may include or be formed from a material comprising the gas permeable body. Owing to the presence of a seal, such masks form part of a closed respiratory support system and are used for delivery of respiratory gases at lower gas flow rates than high flow therapy, in which there is no seal.

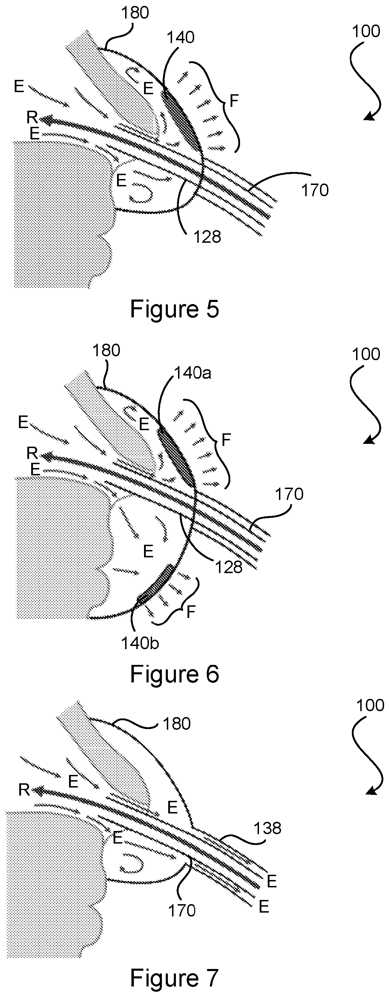

In the embodiment shown in Figure 5, mask 180 covers both nares and nasal elements direct respiratory gas into the patient's airway. Gas permeable body 140 is provided in a portion of mask 180 that is above mask inspiratory lumen 128 which in the example shown, is a nasal prong directing respiratory gas from gas delivery conduit 170 into each of the subject's nares. In one embodiment, mask 180 comprises an outer shell which when applied over the patient's nose and upper lip defines a space around the nasal prongs through which expiratory gases exit the nares and are directed through gas permeable body 140.

In the embodiment shown in Figure 6, mask 180 covers both the nose and mouth and mask inspiratory lumen 128 in the form of nasal prongs directs respiratory gas from conduit 170 into the patient's nares. First gas permeable body 140a is provided in a portion of mask 180 that is above the mask inspiratory lumen 128 and second gas permeable body 140b is provided below the mask inspiratory lumen. The space defined by mask 180 when applied over the nose and mouth enables expiratory gas to be directed through either gas permeable body 140a,b although it may be observed that expiratory gas from the nares is predominantly directed through first gas permeable body 140a and expiratory gas from the mouth is predominantly directed through second gas permeable body 140b.

While particular configurations of mask 180 are illustrated, it is to be understood that the location of gas permeable body 140 in mask 180 is not to be limited to these examples and one or more gas permeable bodies may be provided in any aspect of the mask which provides fluid communication with an expiratory flow path in which expiratory gases E are directed. In some embodiments, the mask 180 may be formed in part or in its entirety from the material that comprises the gas permeable body.

In the embodiment shown in Figure 7, the gas permeable body is located remotely from mask 180. To achieve this, mask expiratory flow lumen 138 directs expiratory gases from the nares (or the nares and mouth if the mask covers both) toward a gas permeable body located remotely from the patient. In one embodiment, mask expiratory lumen 138 is in fluid communication with pores of gas permeable body 140 which is configured as a sheath (not shown) formed around at least part or all of gas delivery conduit 170 in a manner similar to that shown in Figure 4.

It is to be understood that while the mask configurations shown in Figures 5 and 7 have been described in the context of covering both nares, it is contemplated that the mask could alternatively cover a single nare.

An advantage of the embodiment shown in Figure 6 is that expired gases from both the nares and the mouth are directed through the gas permeable body 140 significantly reducing the likelihood of pathogens or other contaminants in the expired gas.

In some embodiments such as those exemplified in Figures 2 to 7 it is envisaged that the gas permeable body 140 comprises a filter material which is porous i.e. permeable to expiratory gases. Thus, "pores" as used throughout the description of the illustrated embodiments refer to the spaces or gaps in the filter material through which expiratory gases travel. In some instances, a porosity or filter material coarseness which allows for normal breathing is sufficient. However, in circumstances where the patient is receiving NHF through patient interface 100, the filter material ideally provides for higher expiratory gas flow rates to avoid compromising treatment or causing harm to the patient. Thus, the filter material is ideally sufficiently coarse to limit the increase in resistance to flow due to expiratory gases being directed through the gas permeable body while also being effective in filtering or intercepting or at the very least reducing the velocity of particles in the expiratory gases. The filter material may include one or more materials selected from a group comprising but not limited to fibrous, porous knitted, mesh, woven foam, textile, non-textile, foam or another filter material.

In some embodiments, a filter material having a larger surface area of filter media may reduce the resistance flow attributable to expiratory gases being directed through the gas permeable body.

### Physical structures

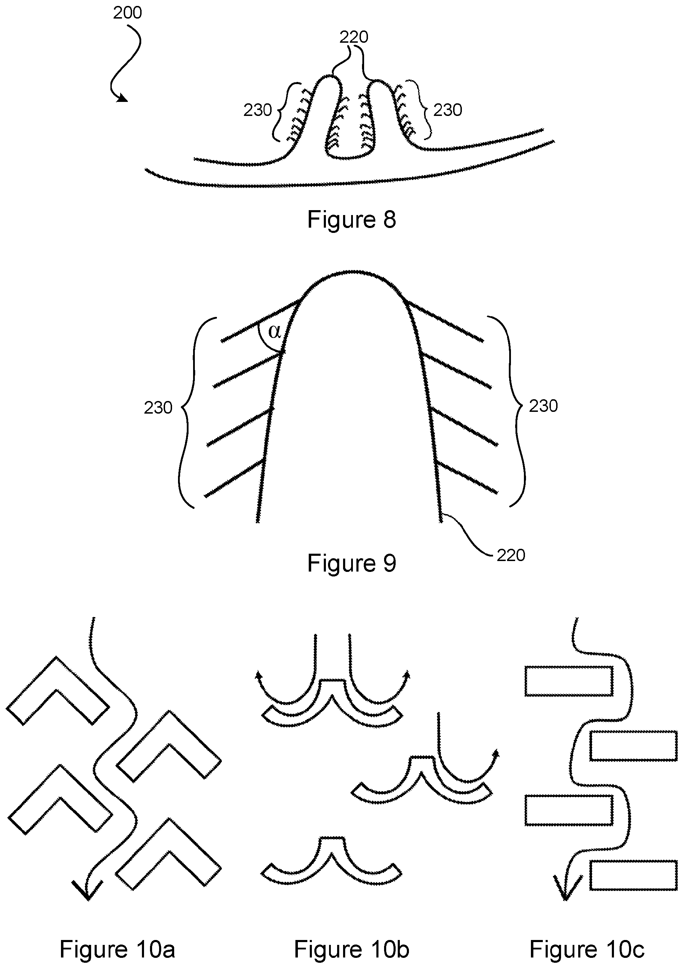

Referring now to Figure 8, there is shown a schematic illustration of patient interface 200 for delivery of gas to a patient through nasal elements 220 in the form of nasal prongs. A plurality of physical structures 230 are provided on an external surface of the nasal prongs and are configured to intercept and/or slow aerosols in expiratory gases from the patient's nare. The physical structures may act to physically disrupt flow of expiratory gases in a straight line out of the nares, such as by creating a tortuous pathway that the expiratory gases and any droplets or particles in them, must travel. The physical structures are configured to increase the likelihood of particles and droplets in the expiratory gases colliding with each other and with the physical structures themselves, thereby increasing droplet size and reducing the risk of airborne transmission of contaminants, e.g. pathogens, which may be contained in the expired gases. Ideally, physical structures 230 are configured to intercept and/or slow aerosols having a size less than 140 µm and preferably, less than 120 µm, e.g. by increasing resistance to flow in the expiratory pathway.

Typically, physical structures 230 extend substantially radially from the surface of the one or more nasal elements 220. Ideally, the physical structures are sufficiently long that they touch or almost touch the internal wall of the patient's nare, although it is envisaged that shorter physical structures would also achieve some level of reduction of transmission risk by disrupting the flow of expiratory gases from the nares and intercepting a portion of the droplets or particles in the expiratory gases, or at least reducing their velocity.

In embodiments where the length of the physical structures is sufficient to at least contact the internal wall of the subject's nares, it may be desirable for the physical structures or an end portion of them to be oriented at an acute angle α

(Figure 9) relative to an outer surface of the nasal element 220 in the direction of insertion into the nare so that insertion is atraumatic to the sensitive tissue inside the nare. The acute angle may be in a range of 10 to 80 degrees.

To accommodate different nostril sizes and to facilitate removal of the patient interface 200 from the nares, one or more of the physical structures may have a flexible portion. For larger nostrils, the physical structures come to rest with a larger angle α, and a smaller angle α in smaller nostrils. Ideally, the physical structures are relatively stiff to substantially maintain their position (and a tortuous expiratory gas flow path) yet may flex or fold (in part) as the nasal prong is withdrawn from the nare so as to avoid trauma to the nasal wall. Thus, some or all of the physical structures may have a flexible portion or are pivotable or towards the attached end that is attached to the nasal element 220.

In some embodiments, such as those shown in Figures 8 and 9, physical structures 230 comprise filaments or whiskers. These may be substantially straight as in Figure 9, or they may have an angled end portion as in Figure 8 or take various other forms. In other embodiments, the physical structures 230 are shaped or configured to at least partly define a tortuous path to intercept and/or slow particles in the expiratory gases. Thus, the shape or configuration of the physical structures may include one or more of, for example, zig-zag, crenelated, coiled/spiral and serpentine. Alternatively or additionally, the physical structures 230 may comprise members formed with a cross-sectional shape configured to enhance the tortuous nature of the expiratory flow path for expiratory gases from the patient's nare. The cross-sectional shape of physical structures 230 may thus include one or more of, for example, chevron (Figure 10a), substantially cornette-shaped (Figure 10b), rectangular (Figure 10c), or triangular, vaulted, V-shaped, W-shaped, roughened and any other closed regular or irregular shape.

To further enhance the tortuous nature of the expiratory flow path, physical structures 230 are ideally provided in a substantially staggered arrangement on the surface of the nasal element. It is contemplated, however, that certain physical structures may still achieve disruption of expiratory flows (and interception and/or slowing of particles) when arranged in line/aligned, staggered, offset, cascading, arranged in a single line wound helically around the nasal element or e.g. arranged in rows along the length of the nasal elements, with each of the physical structures arranged equidistant in the rows and offset from physical structures in adjacent rows. Alternatively, the physical structures may be arranged substantially randomly over the surface of the nasal element.

In some embodiments, the physical structures themselves may have protrusions, for example they may be "hairy", the hairs assisting with capture or slowing of aerosols. The protrusions/hairs increase the surface area of the physical structures, increasing the likelihood of interception of aerosols and/or slowing their exit velocity. The physical structures may be solid, or they may be hollow or include a hollow portion which, depending on their composition, may add to the filtration performance of the structures and/or provide capillary action to draw moisture into the structures themselves.

In some embodiments, some or all of the physical structures have a coating and/or surface modification to improve performance. Coatings that make the physical structures sticky or electrostatic may increase the number of surfaces that intercepted droplets may condense on. Chemical and/or physical changes to the structure or microstructure of the physical structures, or utilising an absorbent material may improve performance. Such changes may include e.g. applying a hydrophilic coating to the physical structures. Surface modifications that increase adhesive capability may include e.g. corona/air plasma treatment which change the surface properties of (e.g. contact angle) of the physical structure, increasing the likelihood of droplets gathering. Corona treatment may render the surface energy of the physical structures higher than that of the liquid, increasing the adhesion of the treated surface.

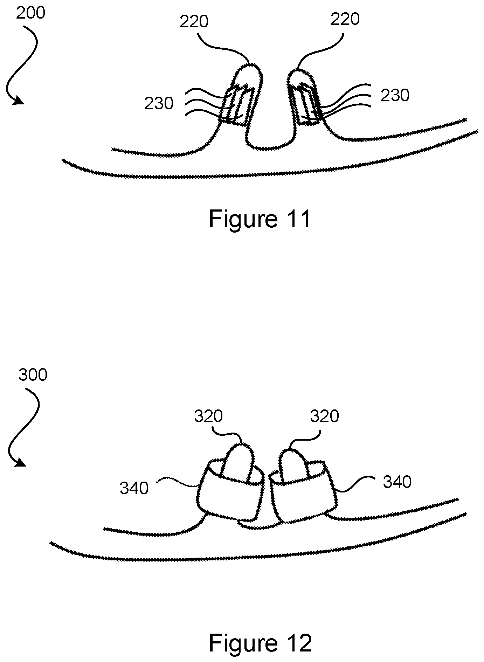

Figure 11 illustrates an embodiment in which the patient interface 200 provides delivery of gas through the patient's nares by nasal elements 220, wherein physical structures 230 are pleats. The pleats may be formed by pinching or folding one or more lengths of a material arranged around the nasal element, increasing the surface area of the material around the nasal element. In other embodiments, the physical structures may be configured as a ruff or relatively stiff frill around the nasal element. In another embodiment, physical structures 230 are fins.

The pleats/fins may be made from a material with absorbent properties that, in addition to slowing flow of expiratory gases, also draw up droplets of moisture. Ideally, the pleats/fins are angled to intercept and/or redirect flow of expiratory gases. The pleats/fins may be oriented on the surface of the nasal element such that they extend radially but need not be orthogonal to the surface. They may be arranged uniformly, equidistant, at uniform angles or at differing spacing and angular orientation with respect to each other and the surface of the nasal element. There may be a single layer of pleats and/or fins provided around the nasal element. Alternatively, there may be several rows of pleats and/or fins provided around the nasal element, which may be offset to reduce direct line of sight past the pleats/fins and into the nare when in situ.

Physical structures 230 may be attached to the external surface of nasal element 220 using any suitable techniques which may involve use of adhesive, bonding or over moulding. In some embodiments, the physical structures are not applied directly to the surface of the nasal element 220 but instead are provided on a substrate which is configured to be applied to an external surface of nasal element 220. The substrate may have an adhesive backing for easy attachment to nasal element 220. Alternatively/additionally the substrate may be configured as a tube-shaped sleeve fixable over the nasal element. The sleeve may be stretchable for placement on and around the nasal element with or without use of adhesive or an adhesive backing.

Figure 12 illustrates another embodiment of the present disclosure in which patient interface 300 comprises least one nasal element 320 in the form of nasal prongs which direct gas into a nare of the patient. A gas permeable body is provided in the form of a substantially cylindrical body 340 positioned around each nasal element 320. Ideally, gas permeable body 340 comprises an open cell foam having a porosity and interconnectedness that enables expiratory gases to easily pass through, whilst filtering contaminant particles. Thus, the open cell foam provides a tortuous pathway for droplets to traverse without significantly increasing resistance to flow which may be particularly beneficial in a patient interface used to deliver NHF.

While the embodiment of Figure 12 shows the gas permeable body as an open cell foam cylinder 340 applied or affixed over the nasal element 320, it is to be understood that in another embodiment, a substantial portion or the entire prong comprising the one or more the nasal elements 320 may be formed from the gas permeable body, such as open cell foam. In this arrangement the nasal element further comprises an internal membrane defining the inspiratory lumen within the nasal element 320. The internal membrane need only be a few µm thick and sufficient to isolate the inspiratory lumen through the nasal elements from the expiratory flow path through the gas permeable body from which the nasal elements are formed.

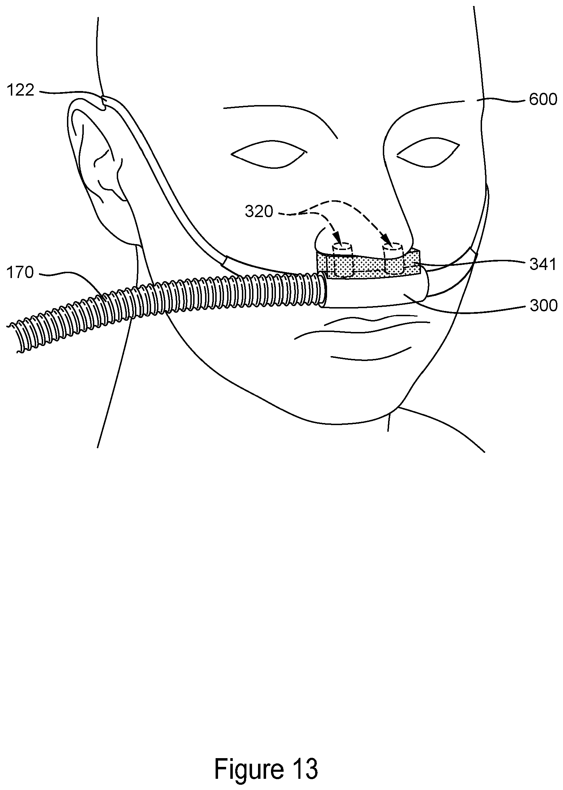

Figure 13 illustrates another embodiment in which patient interface 300 comprises at least one nasal element 320 in the form of nasal prong(s) which direct gas into a nare of the patient. In this embodiment, a gas permeable body is provided in the form of a porous body 341 which substantially surrounds at least the base of the pair of prongs. The porous body 341 is configured to extend along an upper surface of the patient interface 300, underneath the patient nares in use although in some embodiments, part of the porous body 341 may be contoured to assist with location beneath the patient's nares. The porous body 341 may occupy all or part of a space between the patient's nares, and all or part of a space between the upper surface of a manifold portion of the patient interface 300 and the patient's nares, such that an upper surface of porous body 341 is adjacent or touching an underside of the patient nares. The porous body 341 may in use, be configured to substantially cover the openings of the nares.

The porous body 341 may be an open cell foam cushion applied or affixed over the nasal elements 320. Similar to the gas permeable body of Figure 12, the porous body 341 may be comprise a body of open cell foam having a porosity and interconnectedness that enables expiratory gases to pass through, whilst filtering particles in the expiratory gases. The porous body 341 ideally has sufficiently low resistance to flow so as not to substantially raise the pressure in the patient's airways. The porous body 341 may be removable from the patient interface 300 or it may be integral with the patient interface. In some embodiments, the porous body 341 may be provided as a separate part that may be removably fixable or replaceable onto the patient interface. This may be achieved by passing the nasal elements 320 into holes or openings in the porous body 341 prior to applying the patient interface to the patient's face for provision of therapy.

The properties of the open cell foam material proposed in certain embodiments of the disclosure enable the gas permeable body 340 and porous body 341 to act like a sponge, intercepting and/or collecting droplets from expiratory gases thereby reducing transmission risk. The foam material may also act to diffuse expiratory gas flow and decrease velocity of particles in the exhaled gases. In some embodiments, the open cell foam material may act to wick moisture.

Additionally, the resiliently compressible properties of the open cell foam material enable it to be crushed or compressed for insertion into or against the nare, where it expands and conforms to the available space such as the inside contours of the nare, or the space between the upper surface of the patient interface 300 and the patient's nares. Open cell foams can also possess unusual engineering characteristics such as a negative Poisson ratio. This means that when stretched, the material grows thicker rather than thinner. Thus, bending of the prong, when formed from open cell foam, could result in expansion or swelling of the sides which may be beneficial in ensuring a snug fit in the nares further limiting the likelihood of expiratory gases from the nare bypassing the open cell foam gas permeable body.

Features of the various embodiments may have one or more properties that enhance performance of the patient interface 200 in intercepting and/or slowing the velocity of particles in expiratory gases. For example, a plurality of physical structures 230 may be hydrophilic and/or charged and/or dampened prior to use to encourage droplets to attach to the physical structures, as may the gas permeable body 140/340 or porous body 341.

In various embodiments, at least part of the patient interface may be thermally conductive. For example, an inspiratory lumen directing gas into the patient's airway e.g. through nasal elements, may contain a thermally conductive material and is configured to facilitate warming of gas delivered to the patient. One or more of the physical structures 230 or the gas permeable body 140/340 or porous body 341 may be thermally conductive which may reduce resistance to flow though the gas permeable body and/or the physical structures, particularly when they are dampened or become dampened with use.

In various embodiments the patient interface is configured with a channel (not shown) for collection of droplets removed from expiratory gases from the nare. The channel may drain to a chamber in which collected droplets are retained for safe disposal, or the channel may be configured to return collected droplets to the patient. Ideally, collected droplets are directed by gravity flow, although this need not be the case, and droplets may be blown back, e.g. utilising flows from respiratory support, or utilise capillary action. In some embodiments, the channel may be hydrophobic at its surface so that droplets may more easily move along the channel for collection or return to the patient.

It is to be understood that although the present disclosure has provided examples of various embodiments that separately describe the use of a gas permeable body and physical structures treat expiratory gases, it is to be understood that in some embodiments, a patient interface of the present disclosure could utilise one or more a gas permeable bodies together with a plurality of physical structures to treat expiratory gases from the patient in a manner which reduces risk of transmission by pathogens in those gases by filtering the pathogens from the gas, preventing their aerosolization and/or slowing the velocity at which they are released.

Embodiments of the present disclosure have provided examples of various patient interfaces using gas permeable bodies and/or physical structures to treat expiratory gases before exit from the breathing circuit. These may comprise part of or the entirety of the novel patient interface of the present disclosure, or they may be provided as part of an accessory to be fitted or applied to existing patient interfaces such as nasal cannulas, masks, ETTs, tracheostomy tubes or the like.

Thus, in some embodiments, an accessory for a patient interface having a nasal element (such as a nasal prong) for delivery of gas to a patient may comprise a substrate supporting a plurality of physical structures, the substrate being applicable to an external surface of a nasal prong. Ideally, the physical structures are configured to intercept and/or slow aerosols in expiratory gases from the at least one nare e.g. by providing a tortuous expiratory gas flow path. In some embodiments, the plurality of physical structures are arranged to intercept and/or slow aerosols having a size less than 140 µm and preferably, less than 120 µm. Various features, properties and configurations of the physical structures of the accessory may include one or more of those described above in relation, in particular, to the embodiments of Figures 7 to 10. Alternatively/additionally, an open cell foam may be applied to or formed on the substrate akin to the embodiment shown in Figure 11 which would be adhered to or applied over the nasal prong to form a substantially cylindrical shape.

In some embodiments, the substrate is configured to be applied to an external surface of a nasal element/prong by use of an adhesive on the opposing surface of the substrate which may be manufactured with an easily removable backing which when removed, reveals adhesive suitable for attaching the substrate to the external surface of the nasal element. Thus, the substrate may be manufactured with the physical structures applied to its surface in large sheets and cut to size before sterile packaging, or sheets of the substrate with the physical structures applied or formed thereon may be manufactured and sold with the intention that they are cut to size just prior to attachment to nasal prongs. In other embodiments, the substrate comprises a tube-shaped sleeve which may be removably fixable over the nasal element with or without adhesive. In one embodiment, the substrate is configured to be stretched and applied over the nasal element without adhesive.

In other embodiments, an accessory for a patient interface having a nasal element for delivery of gas to a patient, comprises a substantially cylindrical body positioned around the nasal element, the substantially cylindrical body being formed at least in part from material comprising a gas permeable body. Ideally, the gas permeable body comprises an open cell foam. Various features, properties and configurations of the gas permeable body of the accessory include those described above in relation, in particular, to the embodiments in Figure 12.

Embodiments of the disclosure are directed, although not limited, to improvements in patient interfaces for delivering respiratory therapy to patients, which improvements may be targeted at reducing risk of infection of staff and others during treatment of infective patients expiring airborne pathogens. For example, in the case of SARS-CoV-2, respiratory droplets are known to carry a transmission risk. However, the risk of transmission by airborne droplets is not well understood. The present disclosure provides, at the very least, a precautionary improvement to patient interfaces that may be used in the delivery of respiratory therapies (including positive pressure therapies and NHF) that may be perceived to cause aerosolization of particles in expiratory gases particularly for patients known to have infective or transmissible respiratory illness.

Where the terms "comprise", "comprises", "comprised" or "comprising" are used in this specification (including the claims) they are to be interpreted as specifying the presence of the stated features, integers, steps or components, but not precluding the presence of one or more other features, integers, steps or components or group thereof.

It is to be understood that various modifications, additions and/or alterations may be made to the parts previously described without departing from the ambit of the present invention as defined in the claims appended hereto.

## Claims

1. A patient interface for delivery of gas to a patient, the patient interface comprising:
a non-sealing nasal cannula having a pair of nasal prongs (220) configured to direct gas into a nare of the patient; and
a plurality of physical structures (230) on an external surface of at least one of the nasal prongs (220);
an inspiratory conduit (170) for connecting to a gases source for delivering a gases flow to the patient via the non-sealing cannula; and
an associated securement device (112), configured to retain the patient interface on the patient's face in use;
**characterised in that**:
the physical structures extend substantially radially from the surface of the at least one of the nasal prongs (220) and are arranged in a substantially staggered arrangement on the surface of the at least one of the nasal prongs (220) and in rows along a length of the at least one of the nasal prongs (220), to disrupt flow of expiratory gases out of the nares of the patient.

2. The patient interface according to claim 1, wherein the physical structures (230) are configured to provide a tortuous expiratory gas flow path and/or reduce line of sight into the nare when in use.

3. The patient interface according to any one of claims 1 to 2, wherein the physical structures (230) consist of, or comprise a portion that is, oriented at an acute angle relative to an outer surface of the nasal prong (220) in a direction of insertion into the nare.

4. The patient interface according to any one of the preceding claims, wherein at least a plurality of the physical structures (230) have a shape or configuration that at least partly defines a tortuous path and/or reduces line of sight into the nare when in use, to intercept and/or slow aerosols in the expiratory gases.

5. The patient interface according to any one of the preceding claims, wherein at least a plurality of the physical structures (230) have a cross-sectional shape selected from a group comprising but not limited to chevron, vaulted, V-shaped, W-shaped, roughened, substantially cornette-shaped, rectangular, triangular and other regular or irregular closed polygonal shape.

6. The patient interface according to any one of the preceding claims, wherein one or more of the physical structures (230) has at least one end attached to the nasal prong (220).

7. The patient interface according to any one of the preceding claims, wherein one or more of the physical structures (230) is flexible or pivotable at or towards the attached end.

8. The patient interface according to any one of the preceding claims, wherein the one or more physical structures (230) are sufficiently stiff to maintain their position during use.

9. The patient interface according to any one of the preceding claims, wherein the one or more physical structures (230) are or comprise a flexible or pivotable portion.

10. The patient interface according to any one of the preceding claims, wherein the one or more physical structures (230) are solid.

11. The patient interface according to any one of the preceding claims, wherein the one or more physical structures (230) comprise a hollow portion.

12. The patient interface according to claim 9, wherein the physical structures (230) are arranged equidistant in the rows and offset from physical structures in adjacent rows.

## Patentansprüche

1. Patientenschnittstelle zum Abgeben von Gas an einen Patienten, die Patientenschnittstelle umfassend:
eine nicht abdichtende Nasenkanüle, die ein Paar von Nasenstutzen (220) aufweist, das konfiguriert ist, um Gas in ein Nasenloch des Patienten zu leiten; und
eine Vielzahl von physischen Strukturen (230) auf einer Außenoberfläche von mindestens einem der Nasenstutzen (220);
eine Inspirationsleitung (170) zum Anschluss an eine Quelle von Gasen zum Abgeben eines Flusses von Gasen an den Patienten über die nicht abdichtende Kanüle; und
eine zugehörige Befestigungsvorrichtung (112), die konfiguriert ist, um die Patientenschnittstelle auf dem Gesicht des Patienten in Verwendung zu halten;
**dadurch gekennzeichnet, dass:**
sich die physikalischen Strukturen im Wesentlichen radial von der Oberfläche des mindestens einen der Nasenstutzen (220) erstrecken und in einer im Wesentlichen versetzten Anordnung auf der Oberfläche des mindestens einen der Nasenstutzen (220) und in Reihen entlang einer Länge des mindestens einen der Nasenstutzen (220) angeordnet sind, um den Fluss von Ausatemgasen aus den Nasenlöchern des Patienten zu unterbrechen.

2. Patientenschnittstelle nach Anspruch 1, wobei die physischen Strukturen (230) konfiguriert sind, um einen gewundenen Ausatemgasflusspfad bereitzustellen und/oder die Sichtlinie in das Nasenloch in Verwendung zu verringern.

3. Patientenschnittstelle nach einem der Ansprüche 1 bis 2, wobei die physischen Strukturen (230) aus einem Abschnitt bestehen oder einen solchen umfassen, der in einem spitzen Winkel relativ zu einer Außenoberfläche des Nasenstutzens (220) in einer Einführrichtung in das Nasenloch ausgerichtet ist.

4. Patientenschnittstelle nach einem der vorstehenden Ansprüche, wobei mindestens eine Vielzahl der physischen Strukturen (230) eine Form oder Konfiguration aufweisen, die mindestens teilweise einen gewundenen Pfad definiert und/oder die Sichtlinie in das Nasenloch in Verwendung verringert, um Aerosole in den Ausatemgasen abzufangen und/oder zu verlangsamen.

5. Patientenschnittstelle nach einem der vorstehenden Ansprüche, wobei mindestens eine Vielzahl der physischen Strukturen (230) eine Querschnittsform aufweisen, die aus einer Gruppe ausgewählt ist, umfassend, aber nicht beschränkt auf eine zickzackförmige, gewölbte, V-förmige, W-förmige, aufgeraute, im Wesentlichen spitz zulaufende, rechteckige, dreieckige und andere regelmäßige oder unregelmäßige geschlossene polygonale Form.

6. Patientenschnittstelle nach einem der vorstehenden Ansprüche, wobei mindestens eine oder mehrerer der physischen Strukturen (230) mindestens ein Ende aufweist, das an dem Nasenstutzen (220) angebracht ist.

7. Patientenschnittstelle nach einem der vorstehenden Ansprüche, wobei eine oder mehrere der physischen Strukturen (230) an oder zu dem angebrachten Ende hin flexibel oder schwenkbar ist.

8. Patientenschnittstelle nach einem der vorstehenden Ansprüche, wobei die eine oder mehreren physischen Strukturen (230) ausreichend steif sind, um ihre Position während der Verwendung beizubehalten.

9. Patientenschnittstelle nach einem der vorstehenden Ansprüche, wobei die eine oder mehreren physischen Strukturen (230) ein flexibler oder schwenkbarer Abschnitt sind oder einen solchen umfassen.

10. Patientenschnittstelle nach einem der vorstehenden Ansprüche, wobei die eine oder mehreren physischen Strukturen (230) fest sind.

11. Patientenschnittstelle nach einem der vorstehenden Ansprüche, wobei die eine oder mehreren physischen Strukturen (230) einen hohlen Abschnitt umfassen.

12. Patientenschnittstelle nach Anspruch 9, wobei die physischen Strukturen (230) in den Reihen äquidistant und von den physikalischen Strukturen in benachbarten Reihen versetzt angeordnet sind.

## Revendications

1. Interface patient destinée à l'administration de gaz à un patient, l'interface patient comprenant :
une canule nasale non étanche ayant une paire d'embouts nasaux (220) conçues pour diriger le gaz dans le nez du patient ; et
une pluralité de structures physiques (230) sur une surface externe d'au moins une des embouts nasaux (220) ;
un conduit inspiratoire (170) destiné à être raccordé à une source de gaz pour administrer un flux de gaz au patient par l'intermédiaire de la canule non étanche ; et
un dispositif de fixation associé (112), conçu pour maintenir l'interface patient sur le visage du patient en cours d'utilisation ;
**caractérisée en ce que :**
les structures physiques s'étendent sensiblement radialement à partir de la surface de l'au moin un des embouts nasaux (220) et sont disposées sensiblement en quinconce sur la surface de l'au moins un des embouts nasaux (220) et en rangées sur la longueur de l'au moins un des embouts nasaux (220), afin de perturber l'écoulement des gaz expiratoires hors des narines du patient.

2. Interface patient selon la revendication 1, dans laquelle les structures physiques (230) sont conçues pour fournir un chemin d'écoulement de gaz expiratoire tortueux et/ou réduire la ligne de vue dans la narine lors de l'utilisation.

3. Interface patient selon l'une quelconque des revendications 1 à 2, dans laquelle les structures physiques (230) consistent en, ou comprennent une partie qui est orientée à un angle aigu par rapport à une surface extérieure de l'embout nasal (220) dans une direction d'insertion dans la narine.

4. Interface patient selon l'une quelconque des revendications précédentes, dans laquelle au moins une pluralité de structures physiques (230) ont une forme ou une configuration qui définit au moins partiellement un chemin tortueux et/ou réduit la ligne de vue dans la narine lors de l'utilisation, pour intercepter et/ou ralentir les aérosols dans les gaz expiratoires.

5. Interface patient selon l'une quelconque des revendications précédentes, dans laquelle au moins une pluralité de structures physiques (230) ont une forme de section transversale choisie dans un groupe comprenant, mais sans s'y limiter, un chevron, une voûte, une forme en V, une forme en W, une forme rugueuse, une forme sensiblement en cornette, une forme rectangulaire, une forme triangulaire et d'autres formes polygonales fermées régulières ou irrégulières.

6. Interface patient selon l'une quelconque des revendications précédentes, dans laquelle une ou plusieurs des structures physiques (230) ont au moins une extrémité fixée à l'embout nasal (220).

7. Interface patient selon l'une quelconque des revendications précédentes, dans laquelle une ou plusieurs des structures physiques (230) sont flexibles ou pivotantes au niveau de l'extrémité attachée ou vers celle-ci.

8. Interface patient selon l'une quelconque des revendications précédentes, dans laquelle la ou les structures physiques (230) sont suffisamment rigides pour maintenir leur position pendant l'utilisation.

9. Interface patient selon l'une quelconque des revendications précédentes, dans laquelle la ou les structures physiques (230) sont ou comprennent une partie flexible ou pivotante.

10. Interface patient selon l'une quelconque des revendications précédentes, dans laquelle la ou les structures physiques (230) sont solides.

11. Interface patient selon l'une quelconque des revendications précédentes, dans laquelle la ou les structures physiques (230) comprennent une partie creuse.

12. Interface patient selon la revendication 9, dans laquelle les structures physiques (230) sont disposées à équidistance dans les rangées et décalées par rapport aux structures physiques des rangées adjacentes.
